# EUROPEAN PATENT SPECIFICATION

(11) **EP 1 893 318 B1**
(45) Date of publication and mention of the grant of the patent: **22.01.2014**
(21) Application number: 06744330.9
(22) Date of filing: 26.05.2006
(51) Int. Cl.: B01D 46/24, A61M 16/10

(54) **IMPROVEMENTS RELATING TO FILTERS**
VERBESSERUNGEN VON FILTERN
AMELIORATIONS ASSOCIEES A DES FILTRES

(30) Priority: 28.05.2005 GB 0510995
(43) Date of publication of application: 05.03.2008
(73) Proprietor: Intersurgical AG, Vaduz (LI)
(72) Inventor: JASSELL, Surinderjit, Kumar, Berkshire SL4 5RQ (GB); PAYNE, Simon, Robert, Godalming, Surrey GU8 4LU (GB)
(74) Representative: Adamson Jones
(86) International application number: PCT/GB2006/050128
(87) International publication number: WO 2006/129124

(56) References cited:
- EP-A1- 0 167 819
- EP-A2- 0 180 768
- DE-A1- 2 547 857
- GB-A- 2 267 840
- US-A- 4 148 732
- US-A- 5 460 172

## Description

This invention relates to methods of manufacturing filters, and in particular filters for use in artificial respiratory systems.

Filters are devices that prevent or restrict the passage of certain materials, but allow the passage of other materials. Filters are commonly used in artificial respiratory systems, such as anaesthetic or ventilator systems, in order to reduce the risk of microbial cross-contamination. In particular, filters are typically used to protect both the patient from contamination from respiratory apparatus, and the hospital staff from contamination from the exhaust gases of the respiratory circuit. In addition, filters are used in artificial respiratory systems as heat and moisture exchangers that restrict the escape of heat and moisture from the respiratory system.

Conventionally, filters comprise a filter medium that is housed within a filter housing having at least one inlet and at least one outlet. The filter medium is sealed to the interior of the filter housing by a continuous seal, such that gas flowing between the inlet and outlet necessarily flows through the filter medium. A critical factor in the performance of such a filter is the quality of the seal between the filter housing and the filter medium. Any imperfections in this seal may dramatically reduce the efficiency of the filter, and could potentially cause the leakage of fluid from the filter housing during use.

Where the filter medium comprises a web of relatively compressible material, a conventional method of manufacture involves the filter medium being clamped at its periphery between first and second housing components, which are then ultrasonically welded together so as to form a seal between the filter medium and the housing components. However, this method of manufacture is relatively expensive, and it is relatively difficult to achieve a consistent seal of good quality.

Where the filter medium has the form of a preformed filter block, a conventional method of manufacture involves introducing the filter block into a filter housing with a close fit, and applying adhesive so as to form a seal between the filter medium and the filter housing. In order to ensure that gaps between the interior surface of the filter housing and the filter medium are penetrated, and hence an effective seal is obtained, a large quantity of either low viscosity adhesive or reactive expansion adhesive is conventionally used. These techniques therefore require the use of a specialist adhesive in large quantities, and hence are expensive and inconvenient. Furthermore, adhesive is conventionally applied over a peripheral part of an end surface of the filter medium, or alternatively over a peripheral part of an end interior surface of the filter housing, in order to seal the filter medium to the interior surface of the filter housing. This method of manufacture therefore suffers from the major disadvantage that a peripheral part of an end of the filter medium through which gases enter and/or exit the filter medium, during use, is occluded by the adhesive.

One known method of manufacture for this type of filter medium is intended to ensure that adhesive does not occlude a peripheral part of an end of the filter medium through which gases enter and/or exit the filter medium, during use. This method involves a first operation of clamping projecting portions of two opposing side walls of the filter medium between two components of the filter housing, and a second operation of affixing, with adhesive, end components of the filter housing to the two remaining, exposed side walls of the filter medium. Clearly, the need for two separate operations and at least four housing components causes this method to be complicated, and hence expensive, to carry out.

US 5 460 172 discloses a filter for use in an artificial respiratory system.

There has now been devised an improved method of manufacturing a filter which overcomes or substantially mitigates the above-mentioned and/or other disadvantages associated with the prior art.

According to a first aspect of the invention, there is provided a method of manufacturing a filter comprising the steps of
(a) providing a filter medium, and first and second housing components that are engageable with each other so as to define an enclosure for accommodating the filter medium, the first housing component including a continuous seat that extends about the periphery of said enclosure;
(b) introducing a filter medium into the first housing component;
(c) applying a bead of adhesive to either the seat of the first housing component or a continuous part of the filter medium that is adapted to overlie said seat; steps (b) and (c) being performed in any order; and
(d) engaging the first and second housing components together such that pressure is applied to the bead of adhesive, the housing components being adapted such that either application of pressure to the bead of adhesive applied to the seat of the first housing component causes adhesive to be displaced into contact with an adjacent surface of the filter medium, or application of pressure to the bead of adhesive applied to the filter medium causes adhesive to be displaced into contact with an adjacent surface of at least one of the housing components, the adhesive thereby forming a seal between the filter medium and at least one of the housing components.

The method according to this aspect of the invention are advantageous principally because applying pressure to the bead of adhesive enables the adhesive to penetrate small volumes and hence obtain an improved seal. In particular, this feature enables the adhesive to penetrate any irregularities in the surfaces of the housing components, and also any depressions in the filter medium, without the need to use a large amount of low-viscosity adhesive or reactive expansion adhesive. The method of manufacture according to the invention is therefore less complicated and more cost efficient than prior art methods of manufacture. In addition, the method according to this aspect of the invention may be adapted to bond the first housing component to the second housing component in the same operation as that in which a seal is formed between the filter medium and at least one of the housing components, as discussed in more detail below.

Furthermore, the method according to this aspect of the invention is advantageous over ultrasonic welding techniques principally because there is a significant reduction in cost, and also improved consistency and quality of the seal between the housing component(s) and the filter medium. There is therefore less risk of leakage of fluid from the filter during use.

The adhesive is preferably a conventional hot melt glue.

The second housing component preferably applies pressure to the bead of adhesive during engagement of the housing components. This application of pressure may be brought about by the second housing component being urged directly against the bead of adhesive, or alternatively by the second housing component being urged against an intermediate component, such as the filter medium, which is consequently urged against the bead of adhesive.

The adhesive is preferably displaced by the application of pressure into a space between overlapping surfaces of the first and second housing components so as to fix those components together. The first and second housing components are therefore preferably dimensioned such that the space between the first and second housing components into which adhesive is displaced is sufficiently large for adhesive to be able to flow into that space, but also sufficiently small so as to minimise the amount of adhesive required.

In presently preferred embodiments, the first and second housing components each have an enclosure-defining wall with an open end that engages with the corresponding open end of the other housing component, during engagement of the housing components. In particular, the enclosure-defining wall of the first housing component preferably has a connection part adjacent to its open end that receives a corresponding connection part of the enclosure-defining wall of the second housing component, most preferably with a relatively close fit, during engagement of the housing components. In this case, the continuous seat of the first housing component is preferably defined on the interior surface of the enclosure-defining wall, immediately adjacent to the connection part.

A space is preferably defined between overlapping surfaces of the connection parts of the first and second housing components, into which adhesive may be displaced during engagement of the housing components so as to fix those components together. Most preferably, the parts of the overlapping surfaces that are immediately adjacent to the continuous seat, when the housing components have been engaged, include formations that define said space. In particular, the interior surface of the connection part of the first housing component and/or the exterior surface of the connection part of the second housing component may include a circumferential recess that defines said space.

The filter medium may have a generally conventional form. For instance, the filter medium may be formed into a web of relatively compressible material, or the filter medium may be formed into a filter block, which is typically substantially rigid in form. Supplementary filter material that is not sealed to a housing component may also be provided, and this supplementary filter material is preferably accommodated within the first and/or second housing components.

Where the filter medium is formed into a web of relatively compressible material, the filter medium may comprise electrostatically-charged polymeric fibres, or any other suitably compressible filter material. In this case, the second housing component preferably clamps the filter medium against the continuous seat of the first housing component, during engagement of the housing components. During manufacture, the filter medium is preferably positioned so that it is supported at its periphery by the continuous seat, and the bead of adhesive is preferably applied to a continuous part of the filter medium that is adapted to overlie said seat, these steps being performed in any order before engagement of the housing components. Most preferably, the bead of adhesive is applied to a surface of the filter medium that faces away from the continuous seat, such that the bead of adhesive is exposed when the filter medium is being supported by said seat. Alternatively, or in addition, a bead of adhesive may be applied to the continuous seat before the filter medium is introduced into the first housing component. Preferably, adhesive is displaced into a space between overlapping surfaces of the housing components, during engagement of the housing components, so as to fix those components together. Furthermore, the end of the second housing component that clamps the filter medium against the continuous seat is preferably provided with formations that define a recess into which adhesive is displaced, during engagement of the housing components, said adhesive acting to form a seal between the filter medium and the end of the second housing component.

Where the filter medium is formed into a filter block, the filter material may comprise sheet material that is pleated and bonded together, or foam material, or a combination of both. The filter block will typically be either generally cuboidal in form, eg having a square cross-section, or generally cylindrical in form, eg having a circular cross-section. The first housing component is preferably formed such that the continuous seat is situated adjacent to the side wall of the filter medium once the filter medium has been introduced into the first housing component. The bead of adhesive is preferably applied to the continuous seat either before, or after, the filter medium has been introduced into the first housing component. Most preferably, when pressure is applied to the bead of adhesive, adhesive is not displaced over any part of the ends of the filter medium through which gases enter and exit the filter medium during use. The seat is therefore preferably situated adjacent to the side wall of the filter medium, and substantially equidistant from the ends of the filter medium through which gases enter and exit the filter medium during use. Furthermore, the filter medium and the first housing component are preferably dimensioned such that the space between the filter medium and the first housing component is sufficiently large for adhesive to be able to flow into that space, but also sufficiently small so as to minimise the amount of adhesive required. Preferably, the enclosure-defining wall of the first housing component has a holding part of reduced cross-sectional dimensions relative to the connection part. The filter medium is preferably received with a relatively close fit within the holding part, but a space is preferably defined between the interior surface of the connection part and the filter medium. The seat preferably takes the form of a shoulder between the holding part and the connection part of the first housing component. The enclosure-defining wall of the second housing component preferably either abuts the seat, or has a separation from the seat that is filled with adhesive, when the first and second housing components are fully engaged. Most preferably, the interior surface of the enclosure-defining wall of the second housing component aligns with the interior surface of the holding part of the first housing component. In this case, the filter medium is preferably accommodated within the enclosure defined by the first and second housing components with a relatively close fit.

Regardless of the form of the filter medium, each housing component preferably comprises at least one port that enables the inflow and/or outflow of gas during normal use. The filter is preferably adapted for inclusion within an artificial respiratory circuit, and most preferably each of the ports has the form of a standard respiratory apparatus connector.

At least part, and most preferably all, of the first and/or second housing components are preferably translucent so that at least part of the filter medium is visible during use. In order to hide the irregular band of adhesive that may be formed by the method of manufacture according to the invention, an opaque element is preferably formed on the external surface of the first and/or second filter housing components such that the irregular band of adhesive of the assembled filter is hidden from the user.

Hence, in a presently preferred embodiment, the method according to the invention includes the step of injection moulding a second material onto the external surface of one of the housing components, the second material defining an opaque element that at least partially hides the adhesive during use.

This method step is advantageous principally because the provision of an opaque element enables the first and second elements to be at least partially translucent, without the need for a separate component or housing for hiding the adhesive used to fix the first and second elements together. Furthermore, the opaque component may be adapted to form a label, which therefore removes the need for a separate label that might become detached during use, and hence also removes the need for an additional assembly process for affixing the label to the component.

The opaque element and the housing component on which it is formed are preferably formed using a two-shot injection moulding process. This process involves firstly injection moulding the housing component, and subsequently injection moulding the opaque element onto the external surface of the housing component while that component is incompletely cured. This two-shot injection moulding process bonds, and hence securely attaches, the opaque element to the external surface of the housing component.

Alternatively, the opaque element and the housing component on which it is formed may be formed using an overmoulding injection process (also known as insert moulding). This process involves firstly injection moulding the housing component in a first mould, transferring the completely cured housing component to a second mould, and then injection moulding the opaque element onto the external surface of the housing component. This overmoulding injection process bonds, and hence securely attaches, the opaque element to the external surface of the housing component.

At present, however, a two-shot injection moulding process is preferred because it is a simpler process and hence manufacturing costs are reduced. The opaque element is preferably formed of elastomeric material, and most preferably formed of a thermoplastic elastomer (TPE). For both two-shot injection moulding processes and overmoulding injection processes, the material of the translucent housing component and the material of the opaque element are preferably selected so as to form a sufficiently strong and durable bond between those components.

The first and/or second housing components are preferably formed with raised formations in the form of indicia. The indicia are preferably readily visible to a user, and may therefore take the form of an appropriate label. Most preferably, the opaque element is formed about the raised formations so as to provide a background for the indicia.

In presently preferred embodiments, the raised formations are translucent so that the adhesive is visible through those formations. The adhesive may therefore include a colour pigment that contrasts with the colour of the opaque element so as to improve the visibility of the indicia.

The filter may also have a sampling port that enables exhaled gases to be extracted from the filter for testing. In particular, the carbon dioxide levels in a patient's exhaled breath may be tested. The opaque element discussed above has a further advantage in that a closure for the sampling port may be formed integrally with it.

Hence, in a presently preferred embodiment, the method according to the invention includes the following steps for forming the first and second housing components:
(a) moulding the first and second housing components in a first material, at least one of the housing components having a sampling port that enables gas to be extracted, during use, from within the enclosure; and
(b) injection moulding a second material onto the external surface of one of the housing components, the second material including an integral closure for the sampling port.

The second material is preferably inherently more resilient than the first material.

This method forms a filter comprising first and second housing components that define an enclosure for a filter medium, at least one of the housing components having a sampling port that enables gas to be extracted, during use, from within the enclosure, and a second material that has been injection moulded onto the external surface of one of the housing components, the second material having an integral closure for the sampling port.

The filter described above is advantageous principally because the second material, and hence the closure for the sampling port, is bonded directly to a housing component. This feature significantly reduces the risk of the closure becoming detached from the filter housing during use, and may also facilitate a user engaging the closure with, and disengaging the closure from, the sampling port.

Most preferably, the second material is injection moulded onto the external surface of one of the housing components while that component is incompletely cured, in a process commonly known as two-shot injection moulding. This feature generally improves the strength and durability of the bond formed between the housing component and the elastomeric element. Alternatively, one of the housing components is firstly moulded in a first mould, and then transferred to a second mould, into which the second material is injection moulded onto the external surface of that housing component when it has completely cured, in a so-called overmoulding injection process. At present, however, a two-shot injection moulding process is preferred because it is a simpler process and hence manufacturing costs are reduced.

Preferably, the second material includes a connecting arm that projects outwardly, and most preferably substantially perpendicularly, relative to the external surface of the housing component. Most preferably, the connecting arm is sufficiently resilient for it to maintain, substantially, its position relative to the filter housing despite the action of gravity, but also sufficiently flexible to be deformed such that the closure may be engaged with the sampling port. This feature enables a user to readily locate the closure during use.

The sampling port preferably comprises a gas passageway that projects outwardly from a housing component and terminates with an exit orifice. The closure preferably has the form of a cap with an end wall that occludes the exit orifice and a side wall that engages the exterior surface of the sampling port with an interference fit, during use. Most preferably, the closure further includes a plug that extends through the exit orifice and engages the interior surface of the sampling port with an interference fit, during use.

Preferred embodiments of the invention will now be described in greater detail, by way of illustration only, with reference to the accompanying drawings, in which
Figure 1 is a series of cross-sectional views showing three stages of the method according to the invention;
Figure 2 is a perspective view of a first example of a filter manufactured by the method according to the invention;
Figure 3 is a view similar to that of Figure 2, but partly cut-away;
Figure 4 is a perspective view of a second example of a filter manufactured by the method according to the invention;
Figure 5 is a view that is similar to that of Figure 4, but partly cut-away;
Figure 6 is a fragmentary view of the filter of Figures 4 and 5; and
Figure 7 is a perspective view of a third example of a filter manufactured by the method according to the invention.

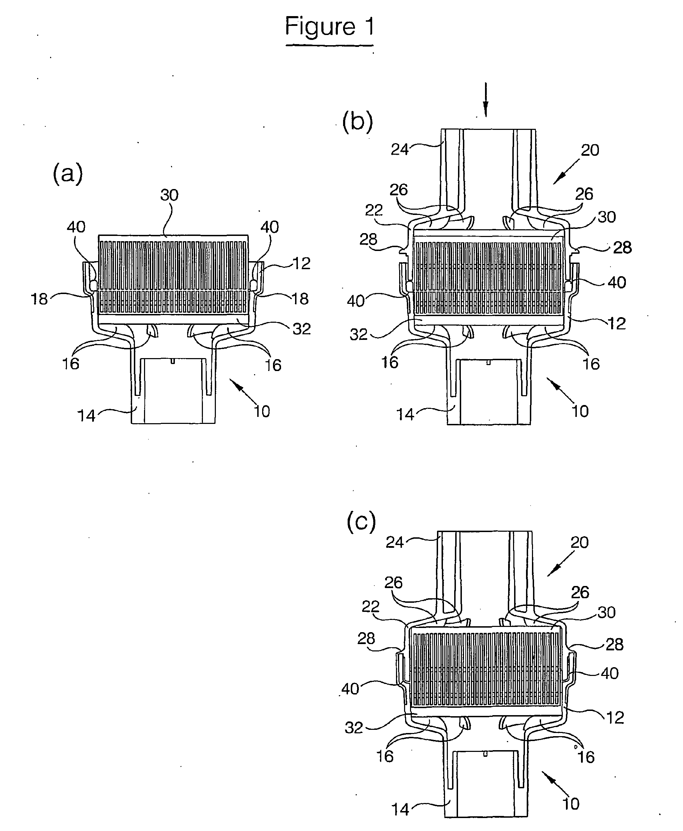

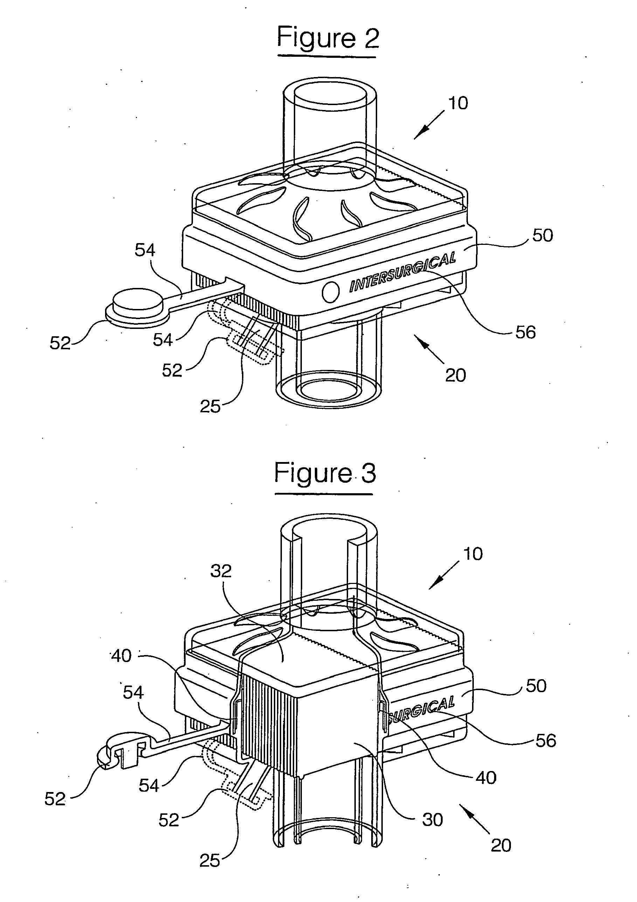

Three stages of a method of manufacturing a filter according to the invention are shown in Figure 1, and a first example of a filter manufactured by that method is shown in Figures 2 and 3.

Before the stages of the method shown in Figure 1 are carried out, a pair of cooperating housing components 10,20, and suitable filter media 30,32 are manufactured or obtained.

The filter media 30,32 may be conventional. In particular, the filter media 30 shown in Figures 1 to 3 comprises a first filter medium 30 having a pleated structure and a second, relatively smaller, filter medium 32 having a foam-like structure. The first filter medium 30 and the second filter medium 32 are not bonded together, but are held alongside one another in the assembled filter. The second filter medium 32 may be a fibre capturing scrim or an HME element.

The first housing component 10 comprises an enclosure-defining wall 12 and a cylindrical connector 14 extending therefrom. The enclosure-defining wall 12 has one open end, and one end from which the connector 14 extends. The connector 14 has the form of a port that enables the inflow and outflow of gas during normal use, and is adapted to connect to standard respiratory apparatus. The connector 14 has a smaller diameter than the enclosure-defining wall 12 such that a shoulder is formed on the interior surface of the first housing component 10 between these two walls 12,14. A series of upstanding ribs 16 are provided on this shoulder so as to space the second filter medium 32 from this shoulder in the assembled filter, and hence ensure that gases flow through the entire filter media 30,32 during use. In addition, the enclosure-defining wall 12 has a connection part adjacent to its open end that has increased internal cross-sectional dimensions relative to the remainder of the enclosure-defining wall 12, such that a continuous shoulder 18 is defined on the interior surface of the enclosure-defining wall 12.

An elastomeric band 50 is formed over the exterior surface of the enclosure-defining wall 12 of the first housing component 10, and includes an integral closure 52 and connecting arm 54, as shown in Figures 2 and 3. The first housing component 10 and elastomeric band 50 are formed together using a so-called two-shot injection moulding process. In particular, the first housing component 10 is injection moulded in a suitably strong and relatively rigid plastics material, such as polypropylene, and then a thermoplastic elastomer (TPE) is injection moulded onto the external surface of the first housing component 10, while it is incompletely cured, so as to form the elastomeric band 50. Since the elastomeric band 50 is moulded directly onto the first housing component 10, these components 10,50 become bonded together.

The second housing component 20 is similar to the first housing component 10, and comprises an enclosure-defining wall 22, a cylindrical connector 24 extending therefrom, and a series of upstanding ribs 26 on the shoulder that is formed on the interior surface of the housing component 20 between these two walls 22,24. The second housing component 20 is injection moulded in a suitably strong and relatively rigid plastics material, such as polypropylene.

In contrast to the first housing component 10, the enclosure-defining wall 22 has a connection part adjacent to its open end that has internal cross-sectional dimensions that are equal to those of the remainder of the enclosure-defining wall 22, such that it fits relatively closely within the connection part of the enclosure-defining wall 12 of the first housing component 10. A small space, however, exists between the connection parts of the first and second housing components 10,20 when the components 10,20 are so engaged. A flange 28 is also provided on the exterior surface of the enclosure-defining wall 22 that is adapted to abut the end of the first housing component 10 when the connection part of the second housing component 20 has been fully received within the connection part of the first housing component 10. In this way, the housing components 10,20 are engaged during manufacture so as to define an enclosure for the filter media 30,32.

In addition, the second housing component 20 is provided with a sampling port 25 (shown in Figures 2 and 3) that enables gases to be extracted from the interior of the enclosure-defining wall 22 of the second housing component 20, during use, for testing. The sampling port 25 is cylindrical in form, and extends outwardly from an opening in the shoulder formed between the enclosure-defining wall 22 and the connector wall 24 of the second housing component 20.

The connecting arm 54 of the elastomeric band 50 extends perpendicularly from the external surface of the first housing component 10, and is adapted to deform such that the closure 52 can be brought into engagement with the sampling port 25, as illustrated in Figures 2 and 3. The closure 52 comprises an end wall for occluding the exit orifice of the sampling port 25, a cylindrical side wall for engaging the exterior surface of the sampling port 25 with an interference fit, and a plug for engaging the interior surface of the sampling port 25 with an interference fit.

Once the first housing component 10, second housing component 20, and filter media 30,32, have been manufactured or obtained, these components 10,20,30,32 are then assembled together as shown in Figure 1, which shows three stages of the method of manufacturing a filter according to the invention. Firstly, the second filter medium 32 is inserted into the enclosure-defining wall 12 of the first housing component 10 with a relatively close fit, such that the second filter medium 32 rests upon the ribs 16 of the first housing component 10. The first filter medium 30 is then inserted into the enclosure-defining wall 12 of the first housing component 10 with a relatively close fit, such that the first filter medium 30 rests upon the exposed surface of the second filter medium 32, and an upper part of the first filter medium 30 projects from the open end of the enclosure-defining wall 12. A small space, however, exists between the side wall of the filter medium 30 and the interior surface of the first housing component 10. A continuous bead of adhesive 40, which is a conventional hot melt glue, is then applied to the shoulder 18 on the internal surface of the enclosure-defining wall 12. This arrangement is shown in Figure 1 a.

The second housing component 20 is then brought into engagement with the first housing component 10, whilst the adhesive 40 is still molten, such that the part of the filter medium 30 that projects from the first housing component 10 is received within the enclosure-defining wall 22. In particular, the connection part of the enclosure-defining wall 22 of the second housing component 20 is received with a relatively close fit, as discussed above, within the connection part of the enclosure-defining wall 12 of the first housing component 10, as shown in Figure 1 b.

As the second housing component 20 is brought into engagement with the first housing component 10, the end surface of the second housing component 20 will come into contact with, and apply pressure to, the bead of adhesive 40 on the shoulder 18 of the first housing component 10. The first and second housing components 10,20 are brought together under sufficient pressure for the bead of adhesive 40 to be compressed by the end surface of the second housing component 20. This action is continued until the flange 28 of the second housing component 20 is brought into abutment with the end of the first housing component 10, as shown in Figure 1c.

As shown in Figure 1 c, compression of the bead of adhesive 40 causes the adhesive 40 to flow into the small space that exists between the connection parts of the first and second housing components 10,20, and also the small space that exists between the side wall of the filter medium 30 and the interior surface of the first and second housing components 10,20. The adhesive 40 situated between the connection parts of the first and second housing components 10,20 acts to fix these components 10,20 together, and the adhesive 40 situated between the side wall of the filter medium 30 and the interior surface of the first and second housing components 10,20 acts to form a seal about the periphery of the filter medium 30.

The application of pressure to the bead of adhesive 40 enables the adhesive to penetrate any irregularities in the surfaces being sealed, and also any depressions in the filter medium 30, so that an improved seal is obtained. The need to use a large amount of low-viscosity adhesive or reactive expansion adhesive is also removed, such that this method of manufacture is less complicated and more cost efficient than prior art methods of manufacture. Furthermore, since the adhesive 40 is situated between the side wall of the filter medium 30 and the interior surface of the first and second housing components 10,20, the adhesive 40 does not extend over an end surface of the filter medium 30 and hence does not restrict the surface area of the filter medium 30 through which gases are able to pass during use.

As shown in Figures 2 and 3, the first and second housing components 10,20 are translucent, and the elastomeric band 50 is opaque. Since the elastomeric band 50 extends over the external surface of the connection part of the first housing component 10, the irregular band of adhesive 40 that is formed between the connection parts of the first and second housing components 10,20 is hidden from the user by the elastomeric band 50.

In addition, the exterior surface of the first housing component 10 is formed with raised formations 56 having the form of indicia. The elastomeric band 50 is moulded onto the exterior surface of the connection part of the first housing component 10 such that an outer surface of each raised formation 56 is exposed and is aligned flush with the exterior surface of the elastomeric band 50. The elastomeric band 50 forms a background for the indicia, such that the indicia are readily visible to a user. This feature removes the need for a separate label that might become detached during use, and hence also removes the need for an additional assembly process for affixing the label to the filter housing.

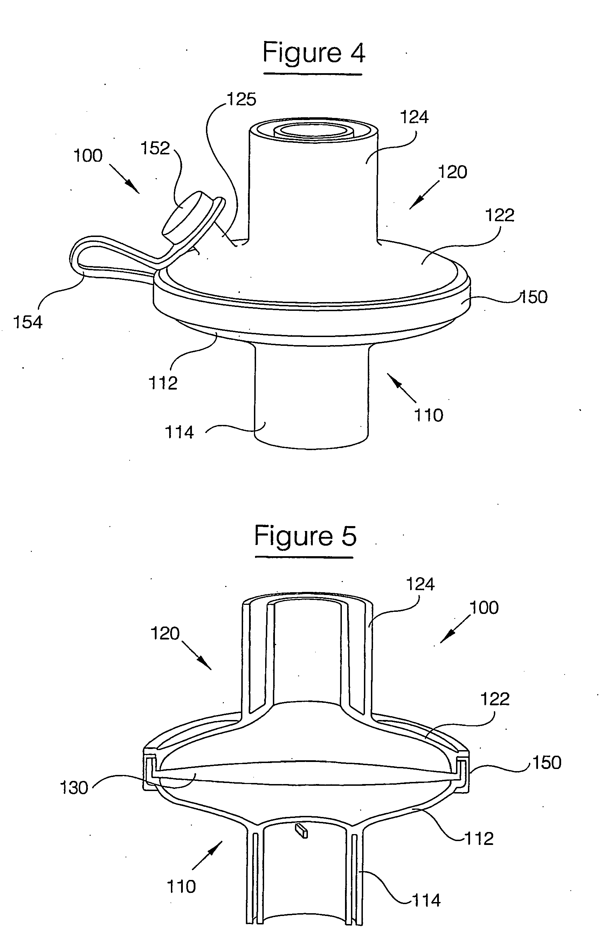

Figures 4 to 6 show a second example of a filter 100 manufactured by the method according to the invention. The filter 100 comprises a pair of cooperating housing components 110,120, and a filter medium 130.

The first housing component 110 comprises an enclosure-defining wall 112 and a cylindrical connector 114 extending therefrom. The enclosure-defining wall 112 has one open end, and one end from which the connector 114 extends. The connector 114 has the form of a port that enables the inflow and outflow of gas during normal use, and is adapted to connect to standard respiratory apparatus.

The enclosure-defining wall 112 has a connection part adjacent to its open end that has increased internal cross-sectional dimensions relative to the remainder of the enclosure-defining wall 112, such that a continuous shoulder 118 is defined on the interior surface of the enclosure-defining wall 112. In addition, an elastomeric band 150 is formed over the exterior surface of the enclosure-defining wall 112 of the first housing component 110, and includes an integral closure 152 and connecting arm 154, as shown in Figure 4. The first housing component 110 and elastomeric band 150 are formed together using a so-called two-shot injection moulding process, as discussed above in relation to the first example of a filter manufactured by the method according to the invention.

The second housing component 120 is similar to the first housing component 110, and comprises an enclosure-defining wall 122, and a cylindrical connector 124 extending therefrom. The enclosure-defining wall 122 has a connection part adjacent to its open end that fits relatively closely within the connection part of the enclosure-defining wall 112 of the first housing component 110. A flange 128 is also provided on the exterior surface of the enclosure-defining wall 122 that is adapted to abut the end of the first housing component 110 when the connection part of the second housing component 120 has been fully received within the connection part of the first housing component 110. In this way, the housing components 110,120 are engaged during manufacture so as to define an enclosure for the filter medium 130.

However, the connection part of the enclosure-defining wall 122 of the second housing component 120 includes a circumferential recess on its exterior surface. In particular, the recess extends from a position a short distance below the flange 128, to the end of the connection part of the second housing component 120. In this way, a small circumferential space exists between the connection parts of the first and second housing components 110,120, in a region that is adjacent to the end of the connection part the second housing component 120, when the components 110,120 are so engaged. The circumferential recess also includes a step so that the recess has a portion of greater depth at the end of the connection part of the second housing component 120.

In addition, the second housing component 120 is provided with a sampling port 125, and associated closure 152 with connecting arm 154, that are essentially identical to those of the first example of a filter manufactured by the method according to the invention, as discussed above.

The filter medium 130 is a cylindrical web of unwoven polymeric fibres that has a diameter substantially equal to that of the interior surface of the connection part of the first housing component 110. In particular, the filter medium 130 is adapted to be received within the connection part of the first housing component 110 with a relatively close fit.

Once the first housing component 110, second housing component 120, and filter medium 130, have been manufactured or obtained, these components 110,120,130 are then assembled together using a method that is similar to that shown in Figure 1. However, the method of assembling this filter 100 differs in a number of respects.

In particular, the filter medium 130 is firstly located within the first housing component 110 such that it is supported at its periphery by the continuous shoulder 130 of that component 110. A continuous bead of adhesive 140, which is a conventional hot melt glue, is then applied to the peripheral portion of the filter medium 140 that is situated above the continuous shoulder 118 on the internal surface of the enclosure-defining wall 112.

The second housing component 120 is then brought into engagement with the first housing component 110, whilst the adhesive 140 is still molten. In particular, the connection part of the enclosure-defining wall 122 of the second housing component 120 is received with a relatively close fit, as discussed above, within the connection part of the enclosure-defining wall 112 of the first housing component 110.

As the second housing component 120 is brought into engagement with the first housing component 110, the end surface of the second housing component 120 will come into contact with, and apply pressure to, the bead of adhesive 140 on the peripheral portion of the filter medium 140 that is situated above the continuous shoulder 118 of the first housing component 110. The first and second housing components 110,120 are brought together under sufficient pressure for the bead of adhesive 140, and also the filter medium 140, to be compressed by the end surface of the second housing component 120. This action is continued until the flange 128 of the second housing component 20 is brought into abutment with the end of the first housing component 110.

Compression of the bead of adhesive 140 causes the filter medium 140 to become clamped between the first and second housing components 110,120, and also causes the adhesive 140 to flow into the small circumferential space that exists between the connection parts of the first and second housing components 110,120, as discussed above. The adhesive 140 situated between the connection parts of the first and second housing components 110,120 acts to fix these components 110,120 together, and the adhesive 140 situated between the filter medium 30 and the second housing component 120 acts to form a seal about the periphery of the filter medium 130.

The application of pressure to the bead of adhesive 140 enables the adhesive to penetrate any irregularities in the surfaces being sealed, and also any depressions in the filter medium 130, so that an improved seal is obtained. Furthermore, this method of manufacture achieves improved consistency and quality of seal between the housing components 110,120 and the filter medium 130 relative to ultrasonic welding techniques. There is therefore less risk of leakage of fluid from the filter during use.

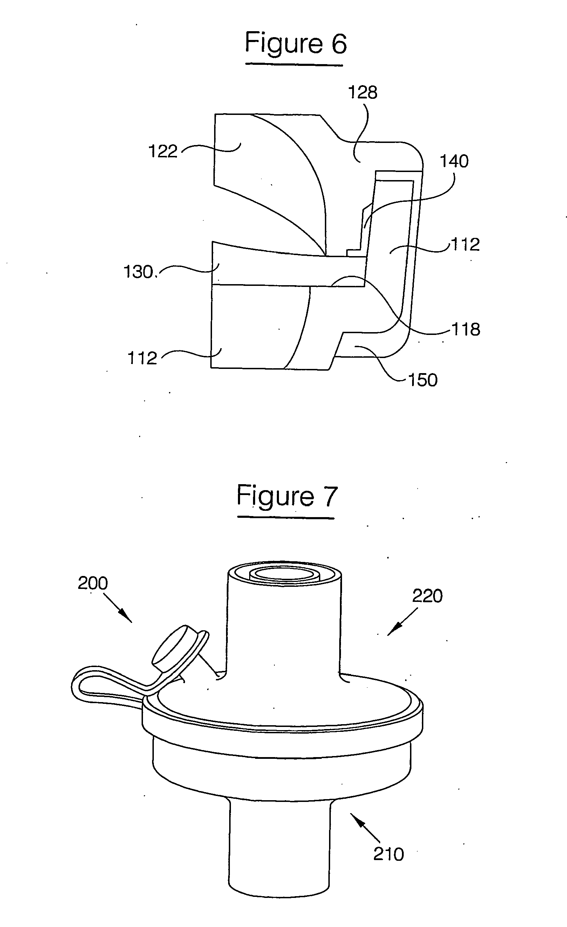

Finally, Figure 7 shows a third example of a filter 200 manufactured by the method according to the invention. This filter 200 has a structure and method of manufacture that is identical to the first example discussed above, save for the housing components 210,220 having a circular, rather than square, cross-sectional shape, and the filter media including a HME element. The HME element acts to restrict the escape of heat and moisture from the respiratory system during use.

## Claims

1. A method of manufacturing a filter adapted for inclusion within an artificial respiratory circuit, the method comprising the steps of
(a) providing a filter medium (30, 130), and first and second housing components (10, 20, 110, 120) that are engageable with each other so as to define an enclosure for accommodating the filter medium, each housing component comprising at least one port that enables the inflow and/or outflow of gas during normal use, each of the ports having the form of a standard respiratory connector (14, 124), and the first housing component including a continuous seat (18, 118) that extends about the periphery of said enclosure;
(b) introducing said filter medium into the first housing component;
(c) applying a bead of adhesive (40, 140) to either the seat of the first housing component or a continuous part of the filter medium that is adapted to overlie said seat; steps (b) and (c) being performed in any order; and
(d) engaging the first and second housing components together such that pressure is applied to the bead of adhesive, the housing components being adapted such that either application of pressure to the bead of adhesive applied to the seat of the first housing component causes adhesive to be displaced into contact with an adjacent surface of the filter medium, or application of pressure to the bead of adhesive applied to the filter medium causes adhesive to be displaced into contact with an adjacent surface of at least one of the housing components, the adhesive thereby forming a seal between the filter medium and at least one of the housing components.

2. A method as claimed in Claim 1, wherein the adhesive is a hot melt glue, and the housing components are injection moulded in plastics material.

3. A method as claimed in Claim 1 or Claim 2, wherein the second housing component applies pressure to the bead of adhesive during engagement of the housing components.

4. A method as claimed in any preceding claim, wherein the adhesive is also displaced by the application of pressure into a space between overlapping surfaces of the first and second housing components so as to fix those components together.

5. A method as claimed in any preceding claim, wherein the first and second housing components each have an enclosure-defining wall (12, 112) with an open end that engages with the corresponding open end of the other housing component, during engagement of the housing components.

6. A method as claimed in Claim 5, wherein the enclosure-defining wall of the first housing component has a connection part adjacent to its open end that receives a corresponding connection part of the enclosure-defining wall of the second housing component, during engagement of the housing components.

7. A method as claimed in Claim 6, wherein the continuous seat of the first housing component is defined on the interior surface of the enclosure-defining wall, immediately adjacent to the connection part.

8. A method as claimed in Claim 6 or Claim 7, wherein a space is defined between overlapping surfaces of the connection parts of the first and second housing components, into which adhesive is displaced during engagement of the housing components so as to fix those components together.

9. A method as claimed in Claim 8, wherein the parts of the overlapping surfaces that are immediately adjacent to the continuous seat, when the housing components have been engaged, include formations that define said space.

10. A method as claimed in Claim 9, wherein the interior surface of the connection part of the first housing component and/or the exterior surface of the connection part of the second housing component include a circumferential recess that defines said space.

11. A method as claimed in any preceding claim, wherein the filter medium is formed into a web of relatively compressible material.

12. A method as claimed in Claim 11, wherein the filter medium comprises electrostatically-charged polymeric fibres.

13. A method as claimed in Claim 12, wherein the second housing component clamps the filter medium against the continuous seat of the first housing component, during engagement of the housing components.

14. A method as claimed in Claim 13, wherein the filter medium is positioned so that it is supported at its periphery by the continuous seat, and the bead of adhesive is applied to a continuous part of the filter medium that is adapted to overlie said seat, these steps being performed in any order before engagement of the housing components.

15. A method as claimed in Claim 14, wherein the bead of adhesive is applied to a surface of the filter medium that faces away from the continuous seat, such that the bead of adhesive is exposed when the filter medium is being supported by said seat.

16. A method as claimed in any one of Claims 13 to 15, wherein a bead of adhesive is applied to the continuous seat before the filter medium is introduced into the first housing component.

17. A method as claimed in any one of Claims 14 to 16, wherein adhesive is displaced into a space between overlapping surfaces of the housing components, during engagement of the housing components, so as to fix those components together.

18. A method as claimed in any one of Claims 14 to 17, wherein the end of the second housing component that clamps the filter medium against the continuous seat is provided with formations that define a recess into which adhesive is displaced, during engagement of the housing components, said adhesive acting to form a seal between the filter medium and the end of the second housing component.

19. A method as claimed in any one of Claims 1 to 10, wherein the filter medium is formed into a filter block.

20. A method as claimed in Claim 19, wherein the filter block is substantially rigid in form.

21. A method as claimed in Claim 19 or Claim 20, wherein the first housing component is formed such that the continuous seat is situated adjacent to the side wall of the filter medium once the filter medium has been introduced into the first housing component.

22. A method as claimed in Claim 21, wherein the bead of adhesive is applied to the continuous seat either before, or after, the filter medium has been introduced into the first housing component.

23. A method as claimed in Claim 21 or Claim 22, wherein the enclosure-defining wall of the first housing component has a holding part of reduced cross-sectional dimensions relative to the connection part, the filter medium being received with a relatively close fit within the holding part, but a space being defined between the interior surface of the connection part and the filter medium.

24. A method as claimed in Claim 23, wherein the seat takes the form of a shoulder (18, 118) between the holding part and the connection part of the first housing component.

25. A method as claimed in Claim 24, wherein the enclosure-defining wall of the second housing component either abuts the seat, or has a separation from the seat that is filled with adhesive, when the first and second housing components are fully engaged.

26. A method as claimed in Claim 25, wherein the interior surface of the enclosure-defining wall of the second housing component aligns with the interior surface of the holding part of the first housing component, such that the filter medium is accommodated within the enclosure defined by the first and second housing components with a relatively close fit.

## Patentansprüche

1. Verfahren zum Herstellen eines Filters zum Einschließen in einen künstlichen Beatmungskreislauf, wobei das Verfahren die folgenden Schritte beinhaltet:
(a) Bereitstellen eines Filtermediums (30, 130) sowie einer ersten und zweiten Gehäusekomponente (10, 20, 110, 120), die miteinander in Eingriff gebracht werden können, um eine Kammer zur Aufnahme des Filtermediums zu definieren, wobei jede Gehäusekomponente wenigstens einen Anschluss aufweist, der das Ein- und/oder Ausströmen von Gas bei normalem Gebrauch ermöglicht, wobei jeder der Anschlüsse die Form eines standardmäßigen Beatmungsverbinders (14, 124) hat und die erste Gehäusekomponente einen fortlaufenden Sitz (18, 118) aufweist, der um die Peripherie der genannten Kammer herum verläuft;
(b) Einführen des genannten Filtermediums in die erste Gehäusekomponente;
(c) Aufbringen einer Klebstoffraupe (40, 140) entweder auf den Sitz der ersten Gehäusekomponente oder auf einen fortlaufenden Teil des Filtermediums, der so gestaltet ist, dass er über dem genannten Sitz liegt; wobei die Schritte (b) und (c) in jeder beliebigen Reihenfolge ausgeführt werden; und
(d) Ineingriffbringen der ersten und zweiten Gehäusekomponente miteinander, so dass Druck auf die Klebstoffraupe aufgebracht wird, wobei die Gehäusekomponenten so ausgelegt sind, dass entweder das Aufbringen von Druck auf die auf den Sitz der ersten Komponente aufgebrachte Gehäuseraupe ein Verdrängen von Klebstoff in Kontakt mit einer Nachbarfläche des Filtermediums bewirkt oder das Aufbringen von Druck auf die auf das Filtermedium aufgebrachte Klebstoffraupe ein Verdrängen von Klebstoff in Kontakt mit einer Nachbarfläche von wenigstens einer der Gehäusekomponenten bewirkt, wobei der Klebstoff so eine Dichtung zwischen dem Filtermedium und wenigstens einer der Gehäusekomponenten bildet.

2. Verfahren nach Anspruch 1, wobei der Klebstoff ein Heißschmelzklebstoff ist und die Gehäusekomponenten aus Plastikmaterial spritzgeformt werden.

3. Verfahren nach Anspruch 1 oder Anspruch 2, wobei die zweite Gehäusekomponente beim Ineingriffbringen der Gehäusekomponenten Druck auf die Klebstoffraupe aufbringt.

4. Verfahren nach einem vorherigen Anspruch, wobei der Klebstoff auch durch das Aufbringen von Druck in einen Raum zwischen überlappenden Flächen der ersten und zweiten Gehäusekomponente verdrängt wird, um diese Komponenten aneinander zu befestigen.

5. Verfahren nach einem vorherigen Anspruch, wobei die erste und zweite Gehäusekomponente jeweils eine kammerdefinierende Wand (12, 112) mit einem offenen Ende aufweisen, das beim Ineingriffbringen der Gehäusekomponenten mit dem entsprechenden offenen Ende der anderen Gehäusekomponente in Eingriff kommt.

6. Verfahren nach Anspruch 5, wobei die kammerdefinierende Wand der ersten Gehäusekomponente ein Verbindungsteil neben ihrem offenen Ende hat, das beim Ineingriffbringen der Gehäusekomponenten ein entsprechendes Verbindungsteil der kammerdefinierenden Wand der zweiten Gehäusekomponente aufnimmt.

7. Verfahren nach Anspruch 6, wobei der fortlaufende Sitz der ersten Gehäusekomponente an der Innenfläche der kammerdefinierenden Wand unmittelbar neben dem Verbindungsteil definiert wird.

8. Verfahren nach Anspruch 6 oder Anspruch 7, wobei ein Raum zwischen überlappenden Flächen der Verbindungsteile der ersten und zweiten Gehäusekomponenten definiert wird, in den beim Eingreifen der Gehäusekomponenten Klebstoff verdrängt wird, um diese Komponenten aneinander zu befestigen.

9. Verfahren nach Anspruch 8, wobei die Teile der überlappenden Flächen, die unmittelbar neben dem fortlaufenden Sitz sind, wenn die Gehäusekomponenten miteinander in Eingriff sind, Formationen aufweisen, die den genannten Raum definieren.

10. Verfahren nach Anspruch 9, wobei die Innenfläche des Verbindungsteils der ersten Komponente und/oder die Außenfläche des Verbindungsteils der zweiten Komponente eine Umfangsaussparung aufweisen, die den genannten Raum definiert.

11. Verfahren nach einem vorherigen Anspruch, wobei das Filtermedium zu einer Bahn aus relativ komprimierbarem Material geformt ist.

12. Verfahren nach Anspruch 11, wobei das Filtermedium elektrostatisch geladene polymere Fasern umfasst.

13. Verfahren nach Anspruch 12, wobei die zweite Gehäusekomponente das Filtermedium beim Eingreifen der Gehäusekomponenten an den fortlaufenden Satz der ersten Gehäusekomponente klammert.

14. Verfahren nach Anspruch 13, wobei das Filtermedium so positioniert wird, dass es an seiner Peripherie von dem fortlaufenden Sitz getragen wird, und die Klebstoffraupe auf einen fortlaufenden Teil des Filtermediums aufgebracht wird, der so ausgelegt ist, dass er über dem genannten Sitz liegt, wobei diese Schritte in einer beliebigen Reihenfolge vor dem Ineingriffbringen der Gehäusekomponenten ausgeführt werden.

15. Verfahren nach Anspruch 14, wobei die Klebstoffraupe auf eine Fläche des Filtermediums aufgebracht wird, die von dem fortlaufenden Sitz weg zeigt, so dass die Klebstoffraupe exponiert wird, wenn das Filtermedium von dem genannten Sitz getragen wird.

16. Verfahren nach einem der Ansprüche 13 bis 15, wobei eine Klebstoffraupe auf den fortlaufenden Sitz aufgebracht wird, bevor das Filtermedium in die erste Gehäusekomponente eingeführt wird.

17. Verfahren nach einem der Ansprüche 14 bis 16, wobei Klebstoff beim Ineingriffbringen der Gehäusekomponenten in einen Raum zwischen überlappenden Flächen der Gehäusekomponenten verdrängt wird, um diese Komponenten aneinander zu befestigen.

18. Verfahren nach einem der Ansprüche 14 bis 17, wobei das Ende der zweiten Gehäusekomponente, die das Filtermedium an den fortlaufenden Sitz klammert, mit Formationen versehen ist, die eine Aussparung definieren, in die beim Ineingriffbringen der Gehäusekomponenten Klebstoff verdrängt wird, wobei der genannte Klebstoff eine Dichtung zwischen dem Filtermedium und dem Ende der zweiten Gehäusekomponente bildet.

19. Verfahren nach einem der Ansprüche 1 bis 10, wobei das Filtermedium zu einem Filterblock geformt wird.

20. Verfahren nach Anspruch 19, wobei der Filterblock im Wesentlichen eine starre Form hat.

21. Verfahren nach Anspruch 19 oder Anspruch 20, wobei die erste Gehäusekomponente so geformt wird, dass sich der fortlaufende Satz neben der Seitenwand des Filtermediums befindet, wenn das Filtermedium in die erste Gehäusekomponente eingeführt wurde.

22. Verfahren nach Anspruch 21, wobei die Klebstoffraupe entweder vor oder nach dem Einführen des Filtermediums in die erste Gehäusekomponente auf den fortlaufenden Sitz aufgebracht wird.

23. Verfahren nach Anspruch 21 oder Anspruch 22, wobei die kammerdefinierende Wand der ersten Gehäusekomponente einen Halteteil mit reduzierten Querschnittsabmessungen relativ zu dem Verbindungsteil hat, wobei das Filtermedium in einer relativ engen Passung in dem Gehäuseteil aufgenommen wird, aber ein Raum zwischen der Innenfläche des Verbindungsteils und dem Filtermedium definiert wird.

24. Verfahren nach Anspruch 23, wobei der Sitz die Form einer Schulter (18, 118) zwischen dem Halteteil und dem Verbindungsteil der ersten Gehäusekomponente einnimmt.

25. Verfahren nach Anspruch 24, wobei die kammerdefinierende Wand der zweiten Gehäusekomponente entweder an den Sitz anstößt oder einen mit Klebstoff gefüllten Abstand von dem Sitz hat, wenn die erste und zweite Komponene völlig im Eingriff miteinander sind.

26. Verfahren nach Anspruch 25, wobei die Innenfläche der kammerdefinierenden Wand der zweiten Gehäusekomponente mit der Innenfläche des Halteteils der ersten Gehäusekomponente fluchtet, so dass das Filtermedium in der durch die erste und zweite Komponente definierten Kammer mit einem relativ engen Sitz aufgenommen wird.

## Revendications

1. Procédé de fabrication d'un filtre adapté pour l'inclusion dans un circuit respiratoire artificiel, le procédé comprenant les étapes consistant à
(a) fournir un milieu filtrant (30, 130), et des premier et second composants de boîtier (10, 20, 110, 120) qui peuvent être mis en prise l'un avec l'autre de manière à définir une enceinte pour accueillir le milieu filtrant, chaque composant de boîtier comprenant au moins un orifice qui permet l'entrée et/ou la sortie de gaz pendant l'utilisation normale, chacun des orifices ayant la forme d'un connecteur respiratoire standard (14, 124), et le premier composant de boîtier incluant un siège continu (18, 118) qui s'étend autour de la périphérie de ladite enceinte ;
(b) introduire ledit milieu filtrant dans le premier composant de boîtier ;
(c) appliquer un cordon d'adhésif (40, 140) soit au siège du premier composant de boîtier, soit à une partie continue du milieu filtrant qui est adaptée pour recouvrir ledit siège ; les étapes (b) et (c) étant effectuées dans n'importe quel ordre ; et
(d) mettre en prise les premier et second composants de boîtier ensemble de telle sorte qu'une pression est appliquée sur le cordon de colle, les composants de boîtier étant adaptés de telle sorte que soit l'application d'une pression sur le cordon d'adhésif appliqué sur le siège du premier composant de boîtier fait que l'adhésif se déplace en contact avec une surface adjacente du milieu filtrant, soit l'application d'une pression sur le cordon d'adhésif appliqué sur le milieu filtrant fait que l'adhésif se déplace en contact avec une surface adjacente d'au moins un des composants de boîtier, l'adhésif formant ainsi un joint d'étanchéité entre le milieu filtrant et au moins un des composants de boîtier.

2. Procédé tel que revendiqué à la Revendication 1, où l'adhésif est une colle thermofusible, et les composants de boîtier sont moulés par injection dans de la matière plastique.

3. Procédé tel que revendiqué à la Revendication 1 ou Revendication 2, où le second composant de boîtier applique une pression sur le cordon d'adhésif pendant la mise en prise des composants de boîtier.

4. Procédé tel que revendiqué dans une quelconque revendication précédente, où l'adhésif est également déplacé par l'application d'une pression dans un espace entre des surfaces se chevauchant des premier et second composants de boîtier de manière à fixer ces composants ensemble.

5. Procédé tel que revendiqué dans une quelconque revendication précédente, où les premier et second composants de boîtier ont chacun une paroi définissant une enceinte (12, 112) avec une extrémité ouverte qui vient en prise avec l'extrémité ouverte correspondante de l'autre composant de boîtier pendant la mise en prise des composants de boîtier.

6. Procédé tel que revendiqué à la Revendication 5, où la paroi définissant une enceinte du premier composant de boîtier a une partie de connexion adjacente à son extrémité ouverte qui reçoit une partie de connexion correspondante de la paroi définissant une enceinte du second composant de boîtier pendant la mise en prise des composants de boîtier.

7. Procédé tel que revendiqué à la Revendication 6, où le siège continu du premier composant de boîtier est défini sur la surface intérieure de la paroi définissant une enceinte, immédiatement adjacente à la partie de connexion.

8. Procédé tel que revendiqué à la Revendication 6 ou Revendication 7, où un espace est défini entre des surfaces se chevauchant des parties de connexion des premier et second composants de boîtier, dans lequel l'adhésif est déplacé pendant la mise en prise des composants de boîtier de manière à fixer ces composants ensemble.

9. Procédé tel que revendiqué à la Revendication 8, où les parties des surfaces se chevauchant qui sont immédiatement adjacentes au siège continu, lorsque les composants de boîtier ont été mis en prise, incluent des formations qui définissent ledit espace.

10. Procédé tel que revendiqué à la Revendication 9, où la surface intérieure de la partie de connexion du premier composant de boîtier et/ou la surface extérieure de la partie de connexion du second composant de boîtier incluent un retrait circonférentiel qui définit ledit espace.

11. Procédé tel que revendiqué dans une quelconque revendication précédente, où le milieu filtrant est formé en une toile de matériau relativement compressible.

12. Procédé tel que revendiqué à la Revendication 11, où le milieu filtrant comprend des fibres polymères chargées électrostatiquement.

13. Procédé tel que revendiqué à la Revendication 12, où le second composant de boîtier serre le milieu filtrant contre le siège continu du premier composant de boîtier pendant la mise en prise des composants de boîtier.

14. Procédé tel que revendiqué à la Revendication 13, où le milieu filtrant est positionné de manière à ce qu'il soit supporté à sa périphérie par le siège continu, et le cordon d'adhésif est appliqué sur une partie continue du milieu filtrant qui est adaptée pour recouvrir ledit siège, ces étapes étant réalisées dans n'importe quel ordre avant la mise en prise des composants de boîtier.

15. Procédé tel que revendiqué à la Revendication 14, où le cordon d'adhésif est appliqué sur une surface du milieu filtrant qui est opposée au siège continu, de telle sorte que le cordon d'adhésif est exposé lorsque le milieu filtrant est supporté par ledit siège.

16. Procédé tel que revendiqué dans une quelconque des Revendications 13 à 15, où un cordon d'adhésif est appliqué au siège continu avant que le milieu filtrant ne soit introduit dans le premier composant de boîtier.

17. Procédé tel que revendiqué dans une quelconque des Revendications 14 à 16, où l'adhésif est déplacé dans un espace entre des surfaces se chevauchant des composants de boîtier, pendant la mise en prise des composants de boîtier, de manière à fixer ces composants ensemble.

18. Procédé tel que revendiqué dans une quelconque des Revendications 14 à 17, où l'extrémité du second composant de boîtier qui serre le milieu filtrant contre le siège continu est pourvue de formations qui définissent un retrait, dans lequel l'adhésif est déplacé pendant la mise en prise des composants de boîtier, ledit adhésif servant à former un joint d'étanchéité entre le milieu filtrant et l'extrémité du second composant de boîtier.

19. Procédé tel que revendiqué dans une quelconque des Revendications 1 à 10, où le milieu filtrant est formé en un bloc filtrant.

20. Procédé tel que revendiqué à la Revendication 19, où le bloc filtrant est de forme substantiellement rigide.

21. Procédé tel que revendiqué à la Revendication 19 ou Revendication 20, où le premier composant de boîtier est formé de telle sorte que le siège continu est situé de manière adjacente à la paroi latérale du milieu filtrant une fois que le milieu filtrant a été introduit dans le premier composant de boîtier.

22. Procédé tel que revendiqué à la Revendication 21, où le cordon d'adhésif est appliqué au siège continu avant ou après que le milieu filtrant a été introduit dans le premier composant de boîtier.

23. Procédé tel que revendiqué à la Revendication 21 ou Revendication 22, où la paroi définissant une enceinte du premier composant de boîtier a une partie de maintien de dimensions transversales réduites par rapport à la partie de connexion, le milieu filtrant étant reçu avec un ajustement relativement serré à l'intérieur de la partie de maintien, mais un espace étant défini entre la surface intérieure de la partie de connexion et le milieu filtrant.

24. Procédé tel que revendiqué à la Revendication 23, où le siège prend la forme d'un épaulement (18, 118) entre la partie de maintien et la partie de connexion du premier composant de boîtier.

25. Procédé tel que revendiqué à la Revendication 24, où la paroi définissant une enceinte du second composant de boîtier soit vient en butée contre le siège, soit comporte une séparation par rapport au siège qui est remplie d'adhésif, lorsque les premier et second composants de boîtier sont pleinement mis en prise.

26. Procédé tel que revendiqué à la Revendication 25, où la surface intérieure de la paroi définissant une enceinte du second composant de boîtier s'aligne sur la surface intérieure de la partie de maintien du premier composant de boîtier, de telle sorte que le milieu filtrant est accueilli dans l'enceinte définie par les premier et second composants de boîtier avec un ajustement relativement serré.
